(19) Europäisches Patentamt

European Patent Office

Office européen des brevets

(11) **EP 1 070 509 A2**

(12) **EUROPEAN PATENT APPLICATION**

(43) Date of publication:
24.01.2001 Bulletin 2001/04

(51) Int. Cl.⁷: **A61M 1/00**

(21) Application number: 00115781.7

(22) Date of filing: 21.07.2000

(84) Designated Contracting States:
**AT BE CH CY DE DK ES FI FR GB GR IE IT LI LU MC NL PT SE**
Designated Extension States:
**AL LT LV MK RO SI**

(30) Priority: 23.07.1999 US 360189

(71) Applicant: **MEDTRONIC, INC.**
**Minneapolis, Minnesota 55432-3576 (US)**

(72) Inventors:
• **Wirth, Lee**
**California 92688 (US)**

• **Elgas, Roger J.**
**Anaheim Hills, California 92802 (US)**
• **Sharareh, Shiva**
**Laguna Hills, California 92653 (US)**

(74) Representative:
**Hughes, Andrea Michelle**
**Frank B. Dehn & Co.,**
**European Patent Attorneys,**
**179 Queen Victoria Street**
**London EC4V 4EL (GB)**

(54) **Safety device for vacuum-assisted venous drainage**

(57) Retrograde blood and/or air flow in the venous line of a vacuum-assisted venous drainage system is avoided by providing a duckbill valve 38 so connected and dimensioned as to limit positive pressure in the airtight venous reservoir 16 to the pressure head of the blood in the venous line, throughout the whole range of clinically expected air flow rates produced by the cardiotomy sucker pumps 24, without however interfering with the vacuum action of the vacuum source.

FIG. 1

EP 1 070 509 A2

**Description**

This invention relates to a safety valve used in connection with a hard-shell venous reservoir using vacuum-assisted venous drainage, and more specifically to a valve for preventing retrograde air flow into the patient's venous system when the vacuum fails.

Heart-lung machines used in open-heart surgery provide an extracorporeal blood circuit that replaces the pumping action of the heart and the oxygenating action of the lungs while the patient's heart is stopped. Blood is drained from the patient's venous system into a venous reservoir, from which it is then pumped through an oxygenator back into the patient's arterial system. During the surgery, an appreciable amount of blood spills into the patient's chest cavity and needs to be removed and recycled. This cardiotomy blood is conventionally sucked up by one or more sucker pumps, filtered and defoamed, and discharged into the venous reservoir for use in the extracorporeal circuit.

Originally, venous blood was simply allowed to run down into the venous reservoir by gravity. Cardiotomy blood was filtered and defoamed in a separate cardiotomy reservoir and then allowed to run down into the venous reservoir. The top of the venous reservoir was open to atmosphere, and the venous reservoir was therefore maintained at atmospheric pressure.

In order to provide adequate flow at the maximum obtainable pressure head dictated by the patient's elevation above the floor, the venous line had to be about 1.3 cm in diameter and about 2-3 m long. That line, and the separate filter-defoamer, represented a considerable priming volume and added significantly to the disposable cost.

For these reasons, perfusionists have in recent years favoured the use of a sealed hard shell combination venous/cardiotomy reservoir which is kept below atmospheric pressure by a vacuum source. The vacuum draws venous blood from the patient, and allows any convenient placement of the reservoir. As a result, the venous line can be as little as 1 m long and 0.65 cm in diameter - a more than 90% saving in venous line priming volume.

The sealed reservoir used in vacuum-assisted venous drainage is subject to variations in internal pressure. The pressure in the reservoir is determined by three factors if roller pumps are used in the equipment. Firstly, the sucker pumps pump blood and/or air into the reservoir and increase the pressure; secondly, the blood pump in the extracorporeal circuit draws blood out of the reservoir and decreases the pressure; and thirdly, the vacuum draws air out of the reservoir and also decreases the pressure.

In normal operation, the perfusionist adjusts these three factors to obtain a balance at which the blood level and the negative pressure in the reservoir stay reasonably constant. Inasmuch as there is always a possibility that human error or equipment failure may cause the positive or negative pressure in the reservoir to exceed design criteria and cause an explosion or implosion of the reservoir, a two-way safety valve is conventionally provided to relieve any dangerous positive or negative pressures. Typically, the safety valve is set to prevent the pressure from exceeding about 16.7-20 kPa (125-150 mmHg) in either direction.

It has recently been found that if, under certain circumstances, the vacuum fails or is inadvertently shut off while the sucker pumps continue to operate at high flow, venous blood in the reservoir is driven back up into the venous line until the blood level in the reservoir falls below the mouth of the venous line. At that point, continued sucker pump operation drives air into the venous line and up into the patient's circulatory system, before the reservoir's safety valves see an overpressure sufficient to vent the reservoir.

Whether or not the above phenomenon occurs depends not so much on the actual pressure in the reservoir as on the rate of air flow created by the sucker pumps. The typical safety valve used on hard shell venous reservoirs limits the reservoir pressure in either direction to very different values for different air flow rates. Therefore, the conventional safety valves will prevent retrograde air injection at a slow air flow rate of, e.g., 0.1 l/min but will fail to prevent it at the more common flow rates of 2 or more l/mm.

The present invention prevents retrograde air injection into the patient's venous system by providing a vacuum-assisted venous drainage system for heart-lung machines, comprising:

a) an airtight reservoir;
b) a venous line connected to drain venous blood into said reservoir at a predetermined pressure head;
c) at least one sucker pump connected to pump cardiotomy blood and air into said reservoir at a rate producing a predetermined air flow rate;
d) a vacuum source connected to evacuate air from said reservoir; and
e) a unidirectional valve so connected to said reservoir as to limit positive air pressure in said reservoir, said valve being arranged to limit said pressure to a value not exceeding said predetermined pressure head throughout the clinically expected range of air flow into said reservoir produced by said sucker pump(s) in the event of failure of said vacuum source. There is a single-direction positive pressure relief valve whose size and characteristics are such that it limits the positive pressure in the reservoir to a value less than the pressure head in the venous line at the maximum clinically expected total air flow rate through the sucker pump(s). This is preferably achieved in the invention by a duckbill valve with appropriate characteristics. The valve can be mounted permanently on the reservoir, or it may advantageously be applied to an external location, such as to a normally capped cardiotomy inlet of the reservoir in place of the cap.

Preferred embodiments will now be described, by way of example only, with reference to the drawings.

Fig. 1 is a schematic view of the environment in which the invention is useful;

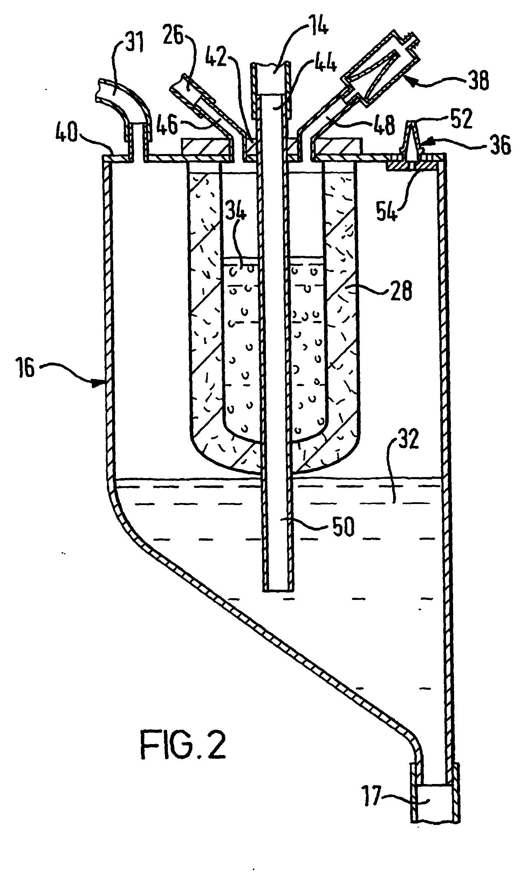

Fig. 2 is a schematic vertical section of a combination venous/cardiotomy reservoir with vacuum assist using the invention;
Fig. 3 is a vertical section of a duckbill valve useful in the invention; and
Fig. 4 is a flow/pressure diagram illustrating the functioning of the safety valves on the reservoir of Fig. 2.

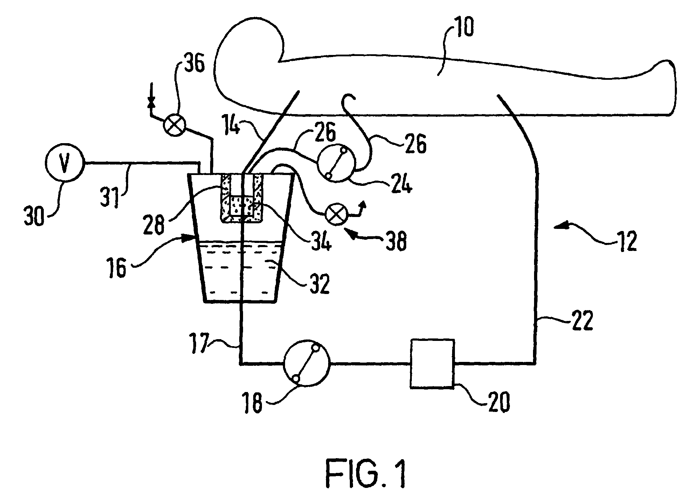

Fig. 1 is a schematic representation of the environment in which the invention operates. A patient 10 is connected during open-heart surgery to an extracorporeal blood circuit 12. Venous blood from the patient 10 drains through a venous line 14 into a reservoir 16. Blood is pumped out of the reservoir 16 through a line 17 by a main pump 18 and is conveyed through an oxygenator 20 into the arterial system of the patient 10 through an arterial line 22. Cardiotomy blood and air are sucked out of the surgical field by one or more sucker pumps 24 through suction lines 26. The lines 26 discharge cardiotomy blood and air into a filter-defoamer 28 which removes the air from the cardiotomy blood and filters out any surgical debris present in the cardiotomy blood. The defoamed and filtered cardiotomy blood is added to the venous blood in the reservoir 16.

In the vacuum assisted mode of venous drainage, a vacuum source 30 is connected by a vacuum line 31 to the top of reservoir 16. The vacuum source 30 draws out the air that fills the space above the venous blood level 32 and the cardiotomy blood level 34. A two-way duckbill-poppet safety valve 36 limits the pressure in the reservoir 16 to a structurally safe valve in both the positive and the negative direction to prevent explosion or implosion of the reservoir 16.

In accordance with the invention, a second safety valve 38 operating only in the positive direction is provided to limit positive pressure in the reservoir 16 to a value below the pressure at which the pressure head in the venous line 14 would be overcome, and a retrograde blood and/or air flow into the patient's venous system would occur. The action of the valve 38 is described in more detail below.

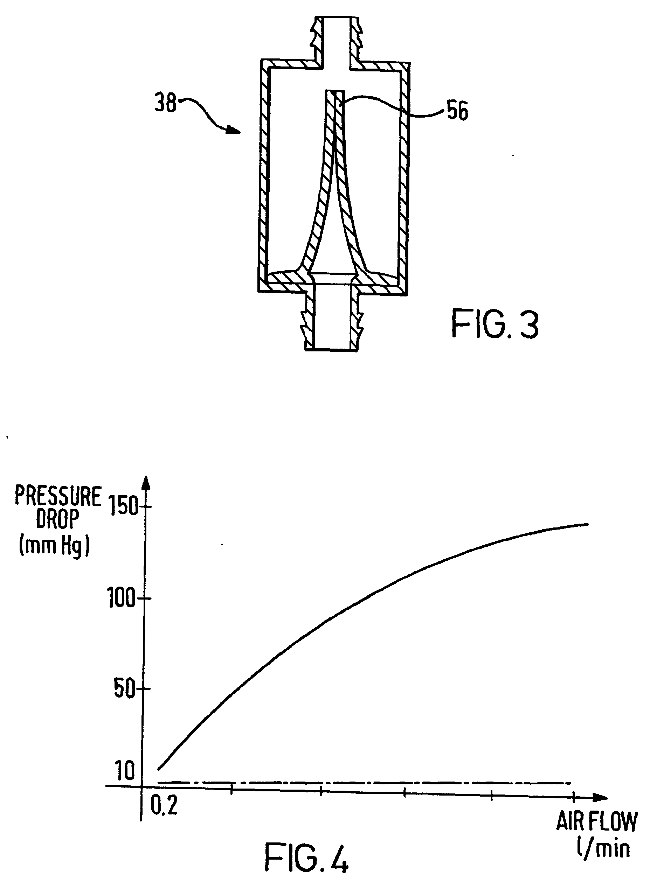

The structure of reservoir 16 is shown in more detail in Fig. 4. The reservoir 16 has an airtight cover 40 on which is mounted a connector turret 42. The turret 42 carries a central connector 44 for the venous line 14 and a number of peripheral connectors 46, 48 for the cardiotomy lines 26. Any unused cardiotomy connectors are capped.

The venous connector 44 connects with a tube 50 whose outlet is below the normal blood level 32 in the reservoir 16. The cardiotomy connector discharges into the top opening of the filter/defoamer cartridge 28. The vacuum line 31 creates a negative pressure on the blood level surface 32. This negative pressure assists in pulling venous blood down from patient 10 through the line 14.

The pressure in the reservoir 16 which draws venous blood from the patient can be expressed as

$$Pr = Pc - Pm - Pv$$

where Pr is the drawing pressure, Pc is the positive pressure produced by the sucker pump 24, Pm is the negative pressure produced by the main pump 18, and Pv is the negative pressure produced by the vacuum source 30. Normally Pr is maintained at a negative pressure not exceeding 8 kPa (60 mmHg).

Because it is possible that one or more of the pumps or the vacuum source may fail, the duckbill-poppet valve 36 is conventionally provided. The duckbill portion 52 of that valve limits the positive pressure in the reservoir 16 to about 20 kPa (150 mmHg), while the poppet portion 54 limits the negative pressure in the reservoir 16 to about the same amount.

The nature of a duckbill valve (shown in more detail in Fig. 3) is that it acts like a variable resistance to air flow through it. Consequently, once a small positive pressure starts to open the duckbill 56, the positive pressure to which it limits the reservoir 16 varies with the amount of air flowing through the duckbill 56. As will be seen from Fig. 4, the duckbill valve 52 of the overpressure safety valve 36 (solid line in Fig. 4) limits the positive pressure in reservoir 16 to about 333.25 Pa (2.5 mmHg) at an air flow rate of 0.05 l/min, but allows it to rise to about 20 kPa (150 mmHg) at a 5 l/mm rate.

At the height with respect to the patient 10 at which the reservoir 16 would normally be placed in a vacuum-assisted venous drainage mode, the pressure head of the venous blood at the exit from tube 50 in the absence of the vacuum would normally be about 240-573 Pa (1.8-4.3 mmHg). It will readily be seen that an inadvertent or accidental shutdown of the vacuum can, at any air flow rate other than a very minimal 0.05-0.1 l/mm, result in retrograde blood flow (and eventually air flow) when the blood level 32 drops below the end of tube 50 into the patient's venous system.

In accordance with the invention, this dangerous condition can be avoided by providing a low-pressure duckbill valve 38 which can be mounted, for example, on an unused cardiotomy connector 48. The characteristics of the valve 38 are preferably such that it limits the pressure in reservoir 16 to about 200 Pa (1.5 mmHg) or less throughout the entire clinically expected air flow range of 0.05-5 l/min, depending upon the number and speed settings of the sucker pumps 24. Duckbill valves having such characteristics include, among others, Model VL4513-103 fluorosilicone valves manufactured by Vernay Laboratories, Inc.

Because the valve 38 is a simple, self-contained duckbill valve, it can be placed not only on an unused cardiotomy connector as described above, but anywhere where its intake is in free-flow relation with the air space in reservoir 16. Also, it thereby lends itself well to distribution as an inexpensive safety accessory to existing airtight hard shell reservoirs. The unidirectional nature of the valve 38 allows it to protect the patient against a vacuum failure without interfering with the normal action of the vacuum source 30 in maintaining a negative pressure in the reservoir 16.

**Claims**

1.  A vacuum-assisted venous drainage system for heart-lung machines, comprising:

    a) an airtight reservoir (16);
    b) a venous line (26) connected to drain venous blood into said reservoir at a predetermined pressure head;
    c) at least one sucker pump (24) connected to pump cardiotomy blood and air into said reservoir at a rate producing a predetermined air flow rate;
    d) a vacuum source (30) connected to evacuate air from said reservoir; and
    e) a unidirectional valve (38) so connected to said reservoir as to limit positive air pressure in said reservoir, said valve being arranged to limit said pressure to a value not exceeding said predetermined pressure head throughout the clinically expected range of air flow into said reservoir (16) produced by said sucker pump(s) (24) in the event of failure of said vacuum source (30)

2.  The system of Claim 1, in which said valve is a duckbill valve (38).

3.  The system of Claim 1 or 2, in which said valve (38) is provided in addition to a bidirectional valve (36) so connected and arranged as to prevent the absolute value of the pressure in said reservoir from exceeding the safe structural limits of said reservoir.

4.  The system of any preceding Claim, in which said valve (38) limits said positive reservoir pressure to less than 200 Pa (1.5 mmHg) at an air flow of 5 l/min.

5.  The system of any preceding Claim, in which said clinically expected air flow range is from 0.05 l/mm to 5 l/min.

6.  The system of any preceding Claim, in which said reservoir includes a plurality of cardiotomy connectors (46, 48) communicating with said reservoir, and said unidirectional valve (38) is connected to one of said cardiotomy connectors in air flow communication therewith.

7.  The system of Claim 6, in which said reservoir includes a filter/defoamer (28), and said cardiotomy connectors (46, 48) communicate with said filter-defoamer.

FIG. 1

EP 1 070 509 A2

FIG.2

FIG. 3

FIG. 4